**Europäisches Patentamt**

**European Patent Office**

**Office européen des brevets**

(19)

(11) Veröffentlichungsnummer: **0 038 919**

**A1**

(12) # EUROPÄISCHE PATENTANMELDUNG

(21) Anmeldenummer: **81101465.3**

(22) Anmeldetag: **28.02.81**

(51) Int. Cl.³: **B 01 J 31/40**
**C 07 C 67/36, C 07 C 69/24**

(30) Priorität: **30.04.80 DE 3016653**

(43) Veröffentlichungstag der Anmeldung:
**04.11.81 Patentblatt 81/44**

(84) Benannte Vertragsstaaten:
**AT BE FR GB IT NL**

(71) Anmelder: **CHEMISCHE WERKE HÜLS AG**
**Postfach 1320**
**D-4370 Marl 1(DE)**

(72) Erfinder: **Müller, Wolfgang Hans Eduard, Dr.**
**Bitterfelder Strasse 9a**
**D-4370 Marl(DE)**

(72) Erfinder: **Hofmann, Peter, Dr.**
**Lipper Weg 193**
**D-4370 Marl(DE)**

(54) **Verfahren zur Rückgewinnung und Reaktivierung von bei der Umsetzung von Olefinen mit Kohlenmonoxid und Alkanolen eingesetzten kobalthaltigen Katalysatoren.**

(57) Verfahren zur Rückgewinnung und Reaktivierung von bei der Umsetzung von Olefinen mit Kohlenmonoxid und Alkanolen eingesetzter Katalysatoren, bestehend aus einer Kobaltverbindung und Pyridin und/oder einem nichtorthosubstituierten Alkylpyridin als Promotor, wobei man das Reaktionsgemisch mit einem sauerstoffhaltigen Gas behandelt, das nicht umgesetzte Alkanol und Olefin, den Promotor sowie die Reaktionsprodukte destillativ entfernt, den dabei anfallenden Destillationsrückstand in Pyridin und/oder einem nichtorthosubstituierten Alkylpyridin aufnimmt und die so erhaltene Suspension mit einem Gemisch aus Kohlenmonoxid und Wasserstoff bei einer Temperatur von 100 bis 250°C und einem Druck von mindestens 50 bar behandelt.

EP 0 038 919 A1

<u>Verfahren zur Rückgewinnung und Reaktivierung von bei der
Umsetzung von Olefinen mit Kohlenmonoxid und Alkanolen
eingesetzter kobalthaltiger Katalysatoren</u>

Es ist bekannt, daß man durch Umsetzung von Olefinen mit
Kohlenmonoxid und H-aciden Komponenten, wie z. B. Alkanolen, in Gegenwart eines Katalysators, der ein Metall der
8. Nebengruppe des Periodischen Systems der Elemente und
gegebenenfalls einen Promotor enthält, Fettsäureester herstellen kann (J. Falbe, Synthesen mit Kohlenmonoxid, Springer-Verlag, Berlin, Heidelberg, New York, 1967).

Eine bevorzugte Variante dieser als Hydrocarboxylierung
bezeichneten Reaktion ist die Umsetzung in Gegenwart von
kobalthaltigen Katalysatoren. Als besonders bevorzugte
Ausführungsform hat sich der zusätzliche Einsatz von Pyridin oder einem nichtorthosubstituierten Alkylpyridin als
Promotor erwiesen.

Ein wesentliches Problem dieser homogen katalysierten Reaktion ist die Rückgewinnung des relativ teuren Kobalts aus
dem Reaktionsgemisch in einer Form, die seinen Wiedereinsatz als Katalysator gestattet.

Ein in DE-OS 19 63 804 beschriebenes Verfahren löst diese
Aufgabe dadurch, daß der kobalthaltige Katalysator als
Destillationssumpf zurückgewonnen und als solcher wieder
in die Reaktion zurückgeführt wird. Ein solches Verfahren
führt jedoch zu Kobaltverlusten infolge Abscheidung metallischen Kobalts bei der destillativen Aufarbeitung.

Gemäß dem Verfahren der US-PS 3 507 891 können solche Verluste vermieden werden, wenn man die kobaltkatalysierte
Hydrocarboxylierung anstelle von Pyridin in Gegenwart eines stabilisierend wirkenden Alkylpyridins durchführt.

In der DE-OS 22 63 907 wird jedoch angeführt, daß auch eine
solche Verfahrensweise weder zu einer vollständigen Kobaltrückgewinnung führt, noch ein durch keinerlei Aktivitätsverluste beeinträchtigtes wiederholtes Recycling des Katalysators ermöglicht. Eine Abscheidung metallischen Kobalts bei der destillativen Aufarbeitung von Hydrocarboxylierungsgemischen wird nach dem Verfahren der DE-OS 22 63 907 dadurch vermieden, daß man die mit Kobalt/Pyridin bzw. Alkylpyridin katalysierte Umsetzung von Olefin mit Alkanol und Kohlenmonoxid in Gegenwart geringer Wassermengen (0,1 bis 2 Mol/Mol Olefin) durchführt. Hierdurch werden neben Estern in geringer Menge die entsprechenden Fettsäuren gebildet. Unter den Bedingungen der destillativen Aufarbeitung von Reaktionsgemischen führt dies zur Bildung fettsauren Kobalts. Die Bildung solcher thermisch stabilen Kobaltverbindungen findet auch statt, wenn man das Reaktionsgemisch vor der Aufarbeitung einer oxidativen Behandlung mit Luft oder Sauerstoff unterwirft.

Ein solches Verfahren ist jedoch nicht ohne Nachteile. So führt die durch die Anwesenheit von Wasser erzwungene Fettsäurebildung zu Ausbeuteverlusten der erwünschten Ester (Rückgang der Esterausbeute z. T. bis auf 67 %). Weiterhin ergeben sich aus der Anwesenheit von Wasser und Fettsäuren unter den drastischen Bedingungen der Hydrocarboxylierungsreaktion Korrosionsprobleme. Auch führt beim Einsatz paraffinverdünnter Olefine, wie sie z. B. durch Chlorierung/Dehydrochlorierung von Paraffinen technisch hergestellt werden, die Anwesenheit von Wasser während der Reaktion zur Entmischung und damit zur Verlangsamung der Reaktion. Schließlich ermöglicht auch diese Verfahrensweise keine vollständige Überführung des eingesetzten Kobalts in das gewünschte fettsaure Salz.

Ein weiteres Verfahren zur Katalysatorrückgewinnung ist in der DE-PS 21 59 139 beschrieben. Dabei arbeitet man in Gegenwart eines Methanolüberschusses als Veresterungskomponente und einer Zugabe von Kohlenwasserstoff zum ausreagierten Reaktionsgemisch. Es kommt so zur Ausbildung zweier Phasen, von denen die untere mindestens 95 % des als Katalysator eingesetzten Kobalts enthält. Die untere das Kobalt in aktiver Form enthaltende Phase kann dann direkt in die Hydrocarboxylierungsreaktion zurückgeführt werden.

Die Nachteile dieses Verfahrens sind einmal die Beschränkung des Verfahrens auf die Herstellung von Methylestern und zum anderen der Verlust des in der oberen Phase verbliebenen Kobalts (bis zu 5 Gewichtsprozent).

Nach dem Verfahren der US-PS 4 041 057 kann das in der oberen Phase gemäß dem Verfahren nach der DE-PS 21 59 139 verbliebene Kobalt dadurch zurückgewonnen werden, daß man den nach destillativer Aufarbeitung der Phase erhaltenen teerartigen Rückstand bei Temperaturen von 1 000 bis 4 000 °F verbrennt, das hierbei gebildete Kobaltoxid aus den Verbrennungsgasen herausfiltert und durch Umsetzung mit Kohlenmonoxid und Wasserstoff in das Kobaltcarbonyl überführt. Letzteres kann wieder in die Hydrocarboxylierung zurückgeführt werden.

Bei einer Kombination der Verfahren nach der DE-PS 21 59 139 und der US-PS 4 041 057 ist zwar eine vollständige Rückgewinnung des Kobalts möglich, die Rentabilität des Gesamtverfahrens wird jedoch durch den Einsatz großer Kohlenwasserstoffmengen, die die destillative Aufarbeitung erheblich belasten, sowie durch eine aufwendige, mehrstufige Rückgewinnung des in der oberen Phase verbliebenen Restkobalts beeinträchtigt.

Aufgabe der vorliegenden Erfindung war es, ein allgemein anwendbares und technisch in einfacher und wirtschaftlicher Weise durchführbares Verfahren zur Rückgewinnung und Reaktivierung von bei der Umsetzung von Olefinen mit Kohlenmonoxid und Alkanolen eingesetzten Katalysatoren, bestehend aus einer Kobaltverbindung und Pyridin und/oder einem nichtorthosubstituierten Alkylpyridin als Promotor, zu entwickeln.

Diese wurde überraschenderweise durch die in den Patentansprüchen beschriebenen Maßnahmen gelöst. Überraschend
deswegen, weil es angesichts der Ausführungen in der US-PS
4 041 057, Spalte 7, Zeilen 60 bis 64, nicht zu erwarten
war, daß durch die Kombination der verfahrenskritischen
Maßnahmen der bei der destillativen Aufarbeitung anfallende kobalthaltige Rückstand direkt wieder in eine aktive
Form überführt werden kann.

Das erfindungsgemäße Verfahren kann im Prinzip bei allen
Hydrocarboxylierungsverfahren zur Herstellung von Fettsäureestern angewandt werden, bei denen ein Katalysator,
bestehend aus einer Kobaltverbindung und Pyridin und/oder
einem nichtorthosubstituierten Alkylpyridin, eingesetzt
wird (z. B. Verfahren gemäß US-PS 3 507 891 und deutscher
Patentanmeldung P 29 12 489.8). So ist vor allem die Wahl
des eingesetzten Olefins unkritisch, d. h., es können sowohl geradkettige oder verzweigte $\alpha$-Olefine als auch Olefine mit innenständiger Doppelbindung eingesetzt werden.
Aber auch Olefine mit mehr als einer Doppelbindung und
solche mit Substituenten, wie z. B. Aryl-, Cyano-, Carboximethyl- und Hydroxylgruppen, sind geeignet.

Im allgemeinen werden Olefine mit 2 bis 40, vorzugsweise
mit 4 bis 20 Kohlenstoffatomen, eingesetzt, die nach bekannten Verfahren des Standes der Technik erhalten werden
können. So können z. B. $\alpha$-Olefine durch Oligomerisierung
von Ethylen nach Ziegler (DE-PSS 878 560 und 11 90 930)
oder durch Wachscracken, Olefine mit innenständiger Doppelbindung durch Dehydrierung oder Chlorierung und anschließender Dehydrochlorierung von Paraffinen (GB-PS
1 037 868) gewonnen werden.

Bei dem zuletzt genannten Verfahren werden in der Regel
Paraffinschnitte, d. h. Gemische unterschiedlicher C-Zahl
eingesetzt, so daß auch die erhaltenen Olefine keine einheitliche C-Zahl aufweisen.

Außerdem kommen in diesen Olefin-Gemischen natürlich alle denkbaren isomeren Formen vor. Neben den reinen - gegebenenfalls substituierten - Olefinen können auch solche mit einem Gehalt an Paraffinen, z. B. bis zu 85 Gewichtsprozent, eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden.

Nicht nur das eingesetzte Olefin, sondern auch die Art des Alkanols, das mit dem Olefin und Kohlenmonoxid umgesetzt wird, ist für das erfindungsgemäße Verfahren unkritisch. Im allgemeinen werden Alkanole mit 1 bis 20, vorzugsweise 1 bis 4 Kohlenstoffatomen, eingesetzt. Typische Vertreter aus der Gruppe der primären Alkanole sind z. B. Methanol, Ethanol, Propanol-(1) und Butanol-(1).

Weiterhin ist es unwesentlich, welche Kobaltverbindung bei der Hydrocarboxylierung verwendet wird. Carbonyle des Kobalts, z. B. Dikobaltoctacarbonyl, sind ebenso geeignet wie carbonsaure Kobaltsalze, wie z. B. Kobaltacetat, Kobaltnaphthenat und Kobalt-2-ethylhexanoat, und Salze des Kobalts mit anorganischen Säuren, wie z. B. Kobaltnitrat und Kobaltsulfat. Vorzugsweise kommen solche carbonsauren Kobaltsalze zum Einsatz, deren Anionen dem Säurerest der bei der Hydrocarboxylierung gebildeten Fettsäureester entsprechen.

Als Promotoren kommen neben Pyridin allein oder im Gemisch alle nichtorthosubstituierten Alkylpyridine, wie z. B. 3- und 4-Picolin, 3,4- und 3,5-Lutidin und 3- und 4-Ethylpyridin infrage.

Schließlich sind die Reaktionsbedingungen, unter denen die Hydrocarboxylierung durchgeführt wird, für das erfindungsgemäße Verfahren nicht von Bedeutung. Im allgemeinen werden Hydrocarboxylierungsverfahren bei Temperaturen von 80 bis 300, vorzugsweise 150 bis 220 $^{\circ}$C, und Kohlenmonoxid-

drucken von 10 bis 800, vorzugsweise 100 bis 300 bar, durchgeführt.

Verfahrenskritisch für das vorliegende Verfahren ist jedoch die oxidative Behandlung des Reaktionsgemisches vor der Rückgewinnung des Kobalts mit Sauerstoff oder einem sauerstoffhaltigen Gas, vorzugsweise Luft, bei Temperaturen von 20 bis 150, vorzugsweise 50 bis 120 $^\circ$C. Diese Behandlung, die bereits in der US-PS 3 507 891, Spalte 4, Zeilen 21 bis 43, und in der nicht zum Stand der Technik gehörenden deutschen Patentanmeldung P 29 12 489.8, Seite 5, Absatz 2, beschrieben ist, wird solange durchgeführt, bis die bei der folgenden destillativen Aufarbeitung zur Abscheidung von metallischem Kobalt führenden Kobaltverbindungen oxidativ zerstört sind.

Bei der sich anschließenden destillativen Aufarbeitung werden nicht umgesetztes Alkanol und Olefin, der Promotor und die Reaktionsprodukte in einem Schritt oder stufenweise bei Sumpftemperaturen bis maximal 350 $^\circ$C abgetrennt. Bevorzugt ist die stufenweise Aufarbeitung, weil man so Fraktionen erhält, die man mit Ausnahme der Fettsäureester an geeigneter Stelle in den Prozeß zurückführen kann.

Der bei der destillativen Aufarbeitung zurückbleibende teerartige Sumpf mit einem Kobaltgehalt von 2 bis 30, vorzugsweise 4 bis 15 Gewichtsprozent, wird in dem als Promotor verwendeten Pyridin oder nichtorthosubstituierten Alkalpyridin aufgenommen. Dies kann z. B. durch Eintragen des bereits bei ca. 50 $^\circ$C fließfähig werdenden Sumpfes in einem mit Promotor beschickten Rührkessel geschehen. Die Menge des Promotors wird so gewählt, daß das für die Hydrocarboxylierungsreaktion erforderliche Promotor/Kobalt-Verhältnis nicht überschritten wird. Eine bevorzugte Ausführungsform des erfindungsgemäßen Verfahren besteht darin, daß man in erster Linie den aus dem Reaktionsgemisch destillativ abgetrennten Promotor zur Aufnahme des kobalthaltigen Rückstandes benutzt und Verluste gegebenenfalls durch Zugabe frischen Promotors ausgleicht.

Für die Behandlung der so erhaltenen Suspension mit dem
Gemisch aus Kohlenmonoxid und Wasserstoff werden im allgemeinen Gasgemische mit 10 bis 90, vorzugsweise 40 bis 60
Volumenprozent Wasserstoff verwendet.

Die Behandlungstemperatur liegt zwischen 100 und 250 $^\circ$C,
vorzugsweise zwischen 120 und 200 $^\circ$C; der Gesamtdruck muß
mindestens 50 bar betragen. Eine obere kritische Grenze
des Gesamtdruckes wird nicht durch den chemischen Ablauf
des Verfahrens, sondern durch die Druckfestigkeit der zur
Verfügung stehenden Apparate bestimmt. Bevorzugt arbeitet
man im Druckbereich von 100 bis 400 bar.

Die Behandlungsdauer läßt sich leicht durch orientierende
Versuche ermitteln. Sie richtet sich in erster Linie nach
den gewählten Temperatur- und Druckbedingungen und sollte
erfahrungsgemäß mindestens 5 Minuten betragen.
Der Erfolg der Behandlung läßt sich einmal daran erkennen,
daß die in der eingesetzten Suspension enthaltenen Feststoffanteile in Lösung überführt wurden, und zum anderen
daran, daß die so erhaltene Katalysatorlösung wieder aktiv
ist.

Die Behandlung der kobalthaltigen Suspension mit dem Gemisch aus Kohlenmonoxid und Wasserstoff kann z. B. in einem Druckgefäß, einer Kaskade von Druckgefäßen oder einem
Rohrreaktor durchgeführt werden.

Das erfindungsgemäße Verfahren wird durch die nachfolgenden Beispiele näher erläutert.

## Beispiel 1

a) In einem 5 1-V4A-Rührautoklaven wird ein Gemisch aus 1 660 g $\alpha$-Dodecen und 640 g Methanol in Gegenwart von 59 g Kobalttridecanoat (Kobaltgehalt 10 %) und 279 g $\gamma$-Picolin mit Kohlenmonoxid (enthält 2 Vol.-% Wasserstoff) bei einem Gesamtdruck von 250 bar und einer Temperatur von 170 °C umgesetzt. Nach einer Stunde wird die Reaktion durch schnelles Abkühlen unterbrochen.

Die gaschromatographische Analyse des Reaktionsgemisches (1. Ansatz) zeigt neben 35 % Dodecen 65 % Reaktionsprodukte, die zu 98 % aus Tridecansäuremethylester mit einem n-Anteil von 83 % bestehen.

Das Reaktionsgemisch wird in einer Rieselsäule (Länge: 200 cm; Durchmesser: 4 cm), die mit 4 x 4 mm-Raschigringen gefüllt ist, bei Normaldruck und 50 °C im Gegenstrom mit Luft behandelt. Pro Stunde werden 2 1 des Reaktionsgemisches von oben zugeführt; der Luftdurchsatz beträgt 100 1/h. Von der Luft mitgerissene organische Bestandteile des Reaktionsgemisches werden am Kopf der Rieselsäule durch einen Kühler kondensiert.

Das so behandelte Reaktionsgemisch wird durch Destillation in eine Methanol-, Olefin-, $\gamma$-Picolin- und Ester-Fraktion zerlegt. Als Sumpfprodukt verbleiben 65 g eines teerartigen Rückstandes, der das eingesetzte Katalysatormetall enthält.

b) Dieser Rückstand wird in 279 g $\gamma$-Picolin (274 g wiedergewonnenes und 5 g frisches $\gamma$-Picolin) aufgenommen und im 5 1-V4A-Rührautoklaven 0,3 Stunden lang bei 200 bar und 170 °C mit Synthesegas (50 Vol.-% Kohlenmonoxid, 50 Vol.-% Wasserstoff) behandelt. Anschließend wird rasch abgekühlt und auf 1 bar entspannt.

Nach Zugabe von 1 660 g $\alpha$-Dodecen und 640 g Methanol zur Katalysatorlösung und dem Aufpressen von 150 bar Kohlenmonoxid (enthält 2 Vol.-% Wasserstoff) wird schnell auf 170 °C erwärmt. Während der Reaktionsdauer von 1 Stunde wird der Gesamtdruck durch Nachpressen von Kohlenmonoxid auf 250 bar gehalten.

Die gaschromatographische Analyse des Reaktionsgemisches (2. Ansatz) zeigt neben 33 % Dodecen 67 % Reaktionsprodukte, die zu 97 % aus Tridecansäuremethylester mit einem n-Anteil von 82,5 % bestehen.

Nach der Behandlung des Reaktionsaustrags mit Luft und der destillativen Abtrennung von Methanol, Olefin, $\gamma$ - Picolin und Ester [gleiche Bedingungen wie in Beispiel 1 a)] erhält man als Sumpfprodukt 67 g eines teerartigen Rückstandes.

c) Der nach der 2. Umsetzung [Beispiel 1 b)] erhaltene Rückstand wird nochmals unter den in Beispiel 1 b) genannten Bedingungen behandelt und anschließend unter den in Beispiel 1 b) genannten Bedingungen zur Synthese von Tridecansäuremethylester eingesetzt.

Die gaschromatographische Analyse des Reaktionsgemisches (3. Ansatz) zeigt neben 34 % Dodecan 66 % Reaktionsprodukte, die zu 97 % aus Tridecansäuremethylester mit einem n-Anteil von 84 % bestehen.

Vergleichsbeispiel A

65 g eines wie in Beispiel 1 a) als Destillationssumpf erhaltenen teerartigen Rückstandes, der das Katalysatormetall enthält, werden - ohne Behandlung mit Synthesegas - unter den Bedingungen von Beispiel 1 a) erneut als Katalysator für die Herstellung von Tridecansäuremethylester eingesetzt.

Die gaschromatographische Analyse des bei Raumtemperatur (20 °C) Feststoffanteile enthaltenden Reaktionsgemisches zeigt neben 45 % Dodecen 55 % Reaktionsprodukte, die zu 97 % aus Tridecansäuremethylester mit einem n-Anteil von 82 % bestehen.

Das Reaktionsgemisch wird wie in Beispiel 1 a) aufgearbeitet und der hierbei anfallende teerartige Rückstand wird erneut - ohne Behandlung mit Synthesegas - als Katalysator eingesetzt. Die unter den Bedingungen von Beispiel 1 a) durchgeführte Umsetzung liefert ein Feststoffanteile enthaltendes Reaktionsgemisch, das laut gaschromatographischer Analyse neben 56 % Dodecen 34 % Reaktionsprodukte enthält, die zu 96 % aus Tridecansäuremethylester mit einem n-Anteil von 83 % bestehen.

Vergleichsbeispiel B

Ein wie in Beispiel 1 a) erhaltenes Reaktionsgemisch wird ohne vorherige Behandlung mit Luft in einer Rieselsäule destillativ aufgearbeitet. Hierbei kommt es zur Ablagerung von Kobalt im Destillationskolben (Kobaltspiegel), und man erhält eine Esterfraktion mit einem Kobaltgehalt von 13 ppm. Der als Destillationsrückstand verbleibende teerartige Sumpf (66 g) wird nach Vorbehandlung mit Synthesegas unter den Bedingungen des Beispiels 1 b) erneut zur Herstellung von Tridecansäuremethylester eingesetzt.

Die gaschromatographische Analyse des Reaktionsgemisches zeigt neben 40 % Dodecen 60 % Reaktionsprodukte, die zu 98 % aus Tridecansäuremethylester mit einem n-Anteil von 82 % bestehen.

Vergleichsbeispiel C

Vergleichsbeispiel B wird wiederholt mit der Ausnahme, daß bei einem Wiedereinsatz des das Katalysatormetall enthaltenden Destillationsrückstandes auf eine Behandlung mit Synthesegas verzichtet wird.

Man erhält ein Reaktionsgemisch, in dem Feststoffanteile suspendiert sind und das laut gaschromatographischer Analyse neben 48 % Dodecen 52 % Reaktionsprodukte enthält, die zu 97 % aus Tridecansäuremethylester mit einem n-Anteil von 84 % bestehen.

Beispiele 2 bis 9

Beispiel 1 wird wiederholt mit der Ausnahme, daß andere Bedingungen für die Behandlung des teerartigen, den Katalysatormetall enthaltenden, Destillationsrückstand mit Kohlenmonoxid/Wasserstoff-Gemischen gewählt werden. Die Ergebnisse sind in Tabelle 1 zusammengefaßt.

Beispiele 10 bis 16

Beispiel 1 a) und b) wird wiederholt mit der Ausnahme, daß andere Bedingungen für die Behandlung der Reaktionsgemische mit sauerstoffhaltigen Gasen gewählt werden. Die Ergebnisse sind in Tabelle 2 zusammengefaßt.

Beispiel 17

Beispiel 1 a) und b) wird wiederholt mit der Ausnahme, daß anstelle von $\alpha$-Dodecen ein statistisches Gemisch linearer Dodecene mit innenständiger Doppelbindung eingesetzt wird und folgende molare Verhältnisse der Einsatzstoffe gewählt werden:

Olefin/Methanol/$\gamma$-Picolin/Kobalt (eingesetzt als 10 %iges Kobaltnaphthenat) = 1/2/0,4/0,04.
Reaktionsergebnisse siehe Tabelle 3.

Beispiel 18

Beispiel 17 wird mit einem statistischen Gemisch linearer Do- und Tridecene (80 Gewichtsprozent $C_{12}$; 20 Gewichtsprozent $C_{13}$) mit innenständiger Doppelbindung ($\alpha$-Anteil

< 1 %) als Einsatzolefin wiederholt.
Reaktionsergebnisse siehe Tabelle 3.

## Beispiel 19

Beispiel 17 wird mit einem statistischen Gemisch linearer Decene und Undecene (10 Gewichtsprozent $C_{10}$; 90 Gewichtsprozent $C_{11}$) mit innenständiger Doppelbindung ($\alpha$ -Anteil < 1 %) wiederholt, das auf 1 Gewichtsteil Olefin-Gemisch 3 Gewichtsteile eines Gemisches aus Decan und Undecan (10 Gewichtsprozent $C_{10}$; 90 Gewichtsprozent $C_{11}$) enthält. Es werden dabei folgende molare Verhältnisse der Einsatzstoffe gewählt:

Olefin (100 %ig)/Methanol/$\gamma$ -Picolin/Kobalt (eingesetzt als 10 %iges Kobaltnaphthenat) = 1/5/1/0,1.
Reaktionsergebnisse siehe Tabelle 3.

## Beispiel 20

Beispiel 17 wird wiederholt mit der Ausnahme, daß Kobaltnaphthenat durch die gleiche Molmenge Koblatacetat ersetzt wird.
Reaktionsergebnisse siehe Tabelle 3.

## Beispiel 21

Beispiel 17 wird wiederholt mit der Ausnahme, daß Kobaltnaphthenat durch die äquimolare Menge Dikobaltoctacarbonyl ersetzt wird.
Reaktionsergebnisse siehe Tabelle 3.

## Beispiel 22

Beispiel 17 wird wiederholt mit der Ausnahme, daß $\gamma$ -Picolin durch die gleiche Molmenge Pyridin ersetzt wird.
Reaktionsergebnisse siehe Tabelle 3.

Tabelle 1

| Bsp. Nr. | Behandlungsbedingungen des Destillations-rückstandes | | | | | Reaktionsergebnisse | | | | | |
| | | | | | | 2. Ansatz | | | 3. Ansatz | | |
| | Druck | Tempe-ratur | Reaktions-dauer | CO/H$_2$-Zusammen-setzung | | Umsatz | Selekti-vität | n-An-teil | Umsatz | Selekti-vität | n-An-teil |
| | bar | °C | h | CO Vol.-% | H$_2$ Vol.-% | % | % | % | % | % | % |
| 2 | 140 | 170 | 0,3 | 50 | 50 | 63 | 98 | 83 | 64 | 97,5 | 83,5 |
| 3 | 280 | 170 | 0,3 | 50 | 50 | 65 | 97,5 | 84 | 64 | 98 | 83 |
| 4 | 200 | 190 | 0,3 | 50 | 50 | 66 | 98,5 | 83 | 65 | 98 | 84 |
| 5 | 200 | 140 | 0,3 | 50 | 50 | 64 | 98 | 84 | 63 | 97 | 83 |
| 6 | 200 | 170 | 0,15 | 50 | 50 | 65 | 98 | 82,5 | 64 | 98,5 | 82,5 |
| 7 | 200 | 170 | 1,0 | 50 | 50 | 63 | 97 | 83 | 63 | 98 | 82,5 |
| 8 | 200 | 170 | 0,3 | 75 | 25 | 67 | 98 | 84 | 65 | 97,5 | 83 |
| 9 | 200 | 170 | 0,3 | 25 | 75 | 65 | 98,5 | 83 | 65 | 98 | 83,5 |

0038919

Tabelle 2

| Bsp. | Behandlungsbedingungen des Reaktionsgemisches | | | | | Reaktionsergebnisse | | |
| Nr. | Druck | Tempe-ratur | Flüssigkeits-durchsatz | Gasdurch-satz | Gaszusammen-setzung | 2. Ansatz | | |
| | | | | | | Umsatz | Selekti-vität | n-An-teil |
| | bar | °C | l/h | l/h | Vol.-% | % | % | % |
| 10 | 1 | 40 | 2 | 100 | Luft | 65 | 98 | 84 |
| 11 | 1 | 80 | 2 | 100 | Luft | 64 | 97 | 83 |
| 12 | 15 | 60 | 2 | 100 | Luft | 64,5 | 97 | 83 |
| 13 | 1 | 60 | 5 | 100 | Sauerstoff | 64 | 98 | 83 |
| 14 | 1 | 60 | 2 | 150 | Luft | 64 | 98,5 | 83,5 |
| 15 | 1 | 60 | 1 | 50 | Luft | 65 | 98 | 83,5 |
| 16 | 1 | 60 | 2 | 100 | Ar (50), $O_2$ (50) | 65 | 97,5 | 84 |

-14-

0038919

Tabelle 3

| Bsp. Nr. | Reaktionsergebnisse des 1. Ansatzes | | | Reaktionsergebnisse des 2. Ansatzes | | |
|---|---|---|---|---|---|---|
| | Umsatz % | Selektivität % | n-Anteil % | Umsatz % | Selektivität % | n-Anteil % |
| 17 | 52 | 97 | 78 | 53 | 96 | 78 |
| 18 | 50 | 96,5 | 77 | 48 | 97 | 78 |
| 19 | 42 | ≦ 97 | 82 | 42 | ≦ 97 | 81,5 |
| 20 | 50 | 97,5 | 79 | 53 | 97 | 78 |
| 21 | 53 | 97 | 77 | 52 | 97 | 78 |
| 22 | 48 | 98 | 73 | 48 | 97,5 | 74 |

- 15 -

**0038919**

O.Z. 3644

Patentansprüche:

1. Verfahren zur Rückgewinnung und Reaktivierung von bei der Umsetzung von Olefinen mit Kohlenmonoxid und Alkanolen eingesetzter Katalysatoren, bestehend aus einer Kobaltverbindung und Pyridin und/oder einem nichtorthosubstituierten Alkylpyridin als Promotor, dadurch gekennzeichnet, daß man das Reaktionsgemisch mit einem sauerstoffhaltigen Gas behandelt, das nicht umgesetzte Alkanol und Olefin, den Promotor sowie die Reaktionsprodukte destillativ entfernt, den dabei anfallenden Destillationsrückstand in Pyridin und/oder einem nichtorthosubstituierten Alkylpyridin aufnimmt und die so erhaltene Suspension mit einem Gemisch aus Kohlenmonoxid und Wasserstoff bei einer Temperatur von 100 bis 250 °C und einem Druck von mindestens 50 bar behandelt.

2. Verfahren nach Anspruch 1, dadurch gekennzeichnet, daß man den durch Destillation aus dem Reaktionsgemisch abgetrennten Promotor bei der Aufnahme des kobalthaltigen Destillationssumpfes wieder einsetzt.

3. Verfahren nach den Ansprüchen 1 und 2, dadurch gekennzeichnet, daß Gemische aus Kohlenmonoxid und Wasserstoff verwendet werden, die 10 bis 90 Volumenprozent Wasserstoff enthalten.

| **EINSCHLÄGIGE DOKUMENTE** | | | KLASSIFIKATION DER ANMELDUNG (Int. Cl ¹) |
|---|---|---|---|
| Kategorie | Kennzeichnung des Dokuments mit Angabe, soweit erforderlich, der maßgeblichen Teile | betrifft Anspruch | |
| | US - A - 2 626 246 (NARAGON)<br>* Spalte 1, Zeile 50 - Spalte 2, Zeile 48; Seite 3, Zeilen 3-21; Ansprüche 1-7 *<br>-- | 1 | B 01 J 31/40<br>C 07 C 67/36<br>69/24 |
| D | US - A - 3 507 891 (HEARNE)<br>* Seite 2, Zeilen 23-59; Seite 4, Zeilen 21-42; Ansprüche 1-8 *<br>-- | 1,2 | |
| D | US - A - 4 041 057 (FENNING)<br>* Zusammenfassung; Seite 1, Zeile 58 - Seite 2, Zeile 68; Seite 8, Zeilen 2-24; Ansprüche 1-7 *<br>-- | 1,2 | RECHERCHIERTE SACHGEBIETE (Int. Cl.³)<br><br>B 01 J 31/40<br>23/94<br>C 07 C 67/36 |
| | US - A - 3 935 228 (KEBLYS)<br>* Zusammenfassung; Seite 1, Zeile 53 - Seite 2, Zeile 58; Ansprüche 1-26 *<br>-- | 1,3 | |
| DEP | EP - A - 0 017 051 (C.W. HÜLS A.G.)<br>* Zusammenfassung; Seite 5, Zeilen 20-38; Seite 12, Zeile 26 - Seite 13, Zeile 2; Ansprüche 1-5 *<br>(Anmeldetag: 14-03-1980; Veröffentlichungstag: 15-10-1980)<br>-- | 1 | KATEGORIE DER GENANNTEN DOKUMENTE |
| DA | US - A - 3 906 016 (HIROSKI ISA)<br>& DE - A - 2 263 907<br>-- ./. | | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |

| Der vorliegende Recherchenbericht wurde für alle Patentansprüche erstellt. | | | &: Mitglied der gleichen Patentfamilie, übereinstimmendes Dokument |
|---|---|---|---|
| Recherchenort<br>Den Haag | Abschlußdatum der Recherche<br>07-08-1981 | Prüfer<br>LO CONTE | |

EPA form 1503.1 06.78

| | Europäisches Patentamt | **EUROPÄISCHER RECHERCHENBERICHT** |

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| DA | <u>US - A - 3 856 832</u> (ETHYL CORP.)<br>& DE - C - 2 159 139 | |
| DA | <u>DE - A - 1 963 804</u> (UNILEVER) | |
| A | <u>US - A - 3 525 762</u> (YATARO ICHI-KAWA) | |

-----

**KLASSIFIKATION DER ANMELDUNG (Int.Cl. 3)**

**RECHERCHIERTE SACHGEBIETE (Int. Cl. 3)**